# EUROPEAN PATENT SPECIFICATION

(11) **EP 3 193 891 B1**
(45) Date of publication and mention of the grant of the patent: **25.12.2019**
(21) Application number: 15843050.4
(22) Date of filing: 26.08.2015
(51) Int. Cl.: A61K 35/15, A61K 35/28, A61K 38/20, A61P 37/06

(54) **IMMUNOMODULATORY CELLS AND IL-25 TO UPREGULATE THE EXPRESSION OF PD-L1**
IMMUNOMODULATORISCHE ZELLEN UND IL-25 ZUM HOCHREGULIEREN DER EXPRESSION VON PD-L1
CELLULES IMMUNOMODULATRICES ET IL-25 POUR LA RÉGULATION À LA HAUSSE DU PD-L1

(30) Priority: 18.09.2014 US 201462052083 P
(43) Date of publication of application: 26.07.2017
(73) Proprietor: National Health Research Institutes, Zhunan Township, Miaoli County 350 (TW)
(72) Inventor: YEN, Linju, Zhunan, Miaoli County 35053 (TW); LIU, Ko-Jiunn, Zhunan, Miaoli County 35053 (TW); SYTWU, Huey-Kang, Zhunan, Miaoli County 35053 (TW)
(74) Representative: Epping - Hermann - Fischer
(86) International application number: PCT/US2015/047025
(87) International publication number: WO 2016/043937

(56) References cited:
- US-A1- 2009 324 609
- US-A1- 2013 058 903
- US-A1- 2013 108 579
- US-A1- 2014 099 254
- YAN-ZHENG GU ET AL: "Different roles of PD-L1 and FasL in immunomodulation mediated by human placenta-derived mesenchymal stem cells", HUMAN IMMUNOLOGY, vol. 74, no. 3, 1 March 2013 (2013-03-01), pages 267-276, XP055268385, US ISSN: 0198-8859, DOI: 10.1016/j.humimm.2012.12.011
- PATRICIA LUZ-CRAWFORD ET AL: "Mesenchymal Stem Cells Repress Th17 Molecular Program through the PD-1 Pathway", PLOS ONE, vol. 7, no. 9, 17 September 2012 (2012-09-17), page e45272, XP055462249, DOI: 10.1371/journal.pone.0045272
- K IWAMURA ET AL: "siRNA-mediated silencing of PD-1 ligands enhances tumor-specific human T-cell effector functions", GENE THERAPY, vol. 19, no. 10, 24 November 2011 (2011-11-24), pages 959-966, XP055063119, ISSN: 0969-7128, DOI: 10.1038/gt.2011.185
- WANG, WB ET AL.: 'Interleukin-25 Mediates Transcriptional Control Of PD-L1 Via STAT3 In Multipotent Human Mesenchymal Stromal Cells (hMSCs) To Suppress Th17 Responses.' STEM CELL REPORTS. vol. 5, no. 3, 08 September 2015, pages 392 - 404, XP055419617

## Description

### CROSS-REFERENCE TO RELATED APPLICATION

This application claims priority to US Provisional Patent Application No. 62/052,083, filed on September 18, 2014.

### BACKGROUND OF THE INVENTION

### 1. Field of the Invention

The present application relates to methods to upregulate an expression of immunomodulatory cells. In particular, the present invention relates to methods to upregulate an expression of immunomodulatory cells through IL-25.

### 2. Description of the Related Art

Multipotent human mesenchymal stromal cells (hMSCs) are somatic progenitors that can be isolated from bone marrow (BM) (Friedenstein, 1976 Friedenstein, A.J., Int. Rev. Cytol. 1976, 47, 327-359.; Pittenger, M.F. et al., Science 1999, 284, 143-147) and many other sites such as adipose tissue, umbilical cord blood, and placenta (Erices et al., 2000 Erices, A. et al., Br. J. Haematol 2000, 109, 235-242; Yen, B.L. et al., Stem Cells 2005, 23, 3-9; Zuk, P.A. et al., Tissue Eng. 2000, 7, 211-228). Previous studies have indicated that hMSCs can differentiate into the paraxial mesodermal lineages of osteoblasts, chondrocytes and adipocytes as well as other non-mesodermal lineages given the right environmental cues (Dominici, M. et al., Cytotherapy 2006, 8, 315-317; Engler, A.J. et al., Cell 2006, 126, 677-689). As such, hMSCs have been widely applied in many clinical trials for regenerative medicine (Giordano, A. et al., J. Cell. Physiol. 2007, 211, 27-35; Hare, J.M. et al., JAMA 2012, 308, 2369-2379). Moreover, hMSCs have been found to have strong immunomodulatory properties which have tremendous therapeutic potential, as evidenced by the numerous clinical trials for immune-related diseases using these versatile progenitor cells (Gebler, A. et al., Trends Mol. Med. 2012, 18, 128-134; Le Blanc, K. et al., Lancet 2008, 371, 1579-1586; Tan, J. et al., JAMA 2012, 307, 1169-1177). hMSCs modulate diverse populations of leukocytes, with the best studied being that towards T lymphocytes, suppressing T effector functions (Bartholomew, A. et al., Exp. Hematol. 2002, 30, 42-48; Di Nicola, M. et al., Blood 2002, 99, 3838-3843; Uccelli, A. et al., Nat. Rev. Immunol. 2008, 8, 726-736). The molecular basis appears to involve both paracrine factors-especially in the human system-including tumor growth factor-β (TGF-β), indoleamine 2,3-dioxygenase (IDO), and prostaglandin E2 (PGE₂), as well as cell surface molecules which engage leukocyte surface receptors (Uccelli, A. et al., Nat. Rev. Immunol. 2008, 8, 726-736; Chen, P.M. et al., J. Biomed. Sci. 2011, 18, 49-59). hMSCs also influence the diversity of CD4 T helper (Th) subset phenotypes, potently skewing Th1 into Th2 cell responses (Aggarwal, S. et al., Blood 2005, 105, 1815-1822; Aksu, A.E. et al., Clin. Immunol. 2008, 127, 348-358) and potentiating induction of regulatory T cells (Tregs), an immunomodulatory population of T cells (Chang, C.J. et al., Stem Cells 2006, 24, 2466-2477; Maccario, R. et al., Haematologica 2005, 90, 516-525; Selmani, Z. et al., Stem Cells 2008, 26, 212-222).

Yan-Zheng Gu et al. (Human Immunology, Vol. 74, No. 3, 2013, pp. 267-276) describe different roles of PD-L1 and FasL in immunomodulation mediated by human placenta-derived mesenchymal stem cells.

Patricia Luz-Crawford et al. (PLoS ONE, Vol. 7, No. 9, 2012, pp e45272) describe: Mesenchymal Stem Cells Repress Th17 Molecular Program through the PD-1 Pathway.

K Iwamura et al. (Gene Therapy, Vol. 19, No. 10, 2011, pp. 959-966) describe siRNA-mediated silencing of PD-1 ligands enhances tumor-specific human T-cell effector functions.

One population of T lymphocytes which has moved into greater prominence are interleukin (IL)-17A secreting T cells (Dong, 2008). Known also as Th17 cells, this T helper cell subpopulation is important in mediating host responses towards microbial infections, as well as participating in the pathogenesis of many autoimmune and chronic inflammatory diseases that had been long believed to be caused by Th1 cells (Miossec, P. et al., Nat. Rev. Drug Discov. 2012, 11, 763-776). While some studies have shown that hMSCs attenuate Th17-mediated immunity (Ghannam, S. et al., J. Immunol. 2010, 185, 302-312; Gonzalez, M.A. et al., Arthritis Rheum. 2009, 60, 1006-1019; Xu, J. et al., Blood 2012, 120, 3142-3151), others have found that hMSCs actually enhance Th17 responses (Darlington, P.J. et al., Ann. Neurol. 2010, 68, 540-545; Tso, G.H. et al., Stem Cells 2010, 28, 939-954). These discrepant reports are likely due to an incomplete understanding currently of the mechanisms involved in hMSC-Th17 lymphocyte interactions, which have important implications in the clinical use of hMSCs given the role of Th17 cells in human diseases (Korn, T. et al., Annu. Rev. Immunol. 2009, 27, 485-517). It is therefore set out to examine the nature of hMSC-Th17 interactions, and elucidate the mechanisms involved. It was found that hMSCs suppress Th17 responses through both paracrine and cell-cell contact mechanisms, involving IL-25-also known as IL17E-as well as PD-L1, a ligand of the PD-1 family. The data demonstrate that hMSCs constitutively secrete IL-25 to upregulate the cell surface expression of PD-L1 through JNK and STAT3, with STAT3 involved in the transcriptional control of PD-L1. hMSCs has also been described to increased the immunodmodulatory leukocyte population of myeloid-derived suppressor cells (MDSCs) (Yen, B.L. et al, Stem Cell Rep. 2013, 1, 139-51).

### SUMMARY

The present application provides a method of upregulating an expression of immunomodulatory cells in vitro comprising treating the immunomodulatory cells selected from the group consisting of human monocytes and human mesenchymal stromal cells (hMSCs) with IL-25 to increase an expression of PD-L1. The method can further comprise treating the immunomodulatory cells with the JNK inhibitor SP600215 and/or the STAT3 inhibitor WP1066.

The present application also provides immunomodulatory cells selected from the group consisting of human monocytes and human MSCs, and IL-25, for use in the treatment of immune disorders including, but not limited, autoimmune diseases, transplantation, and inflammatory diseases.

### BRIEF DESCRIPTION OF THE DRAWINGS

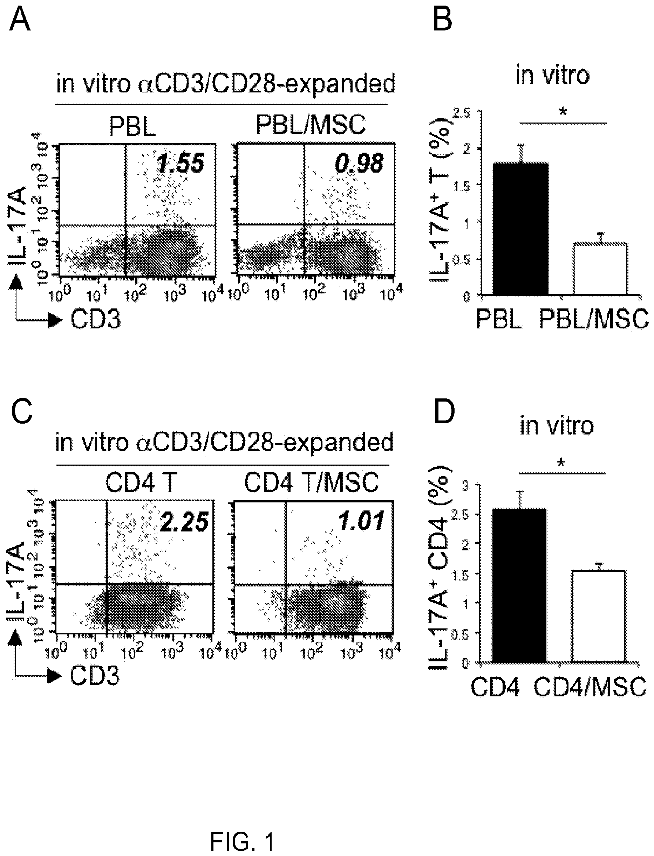

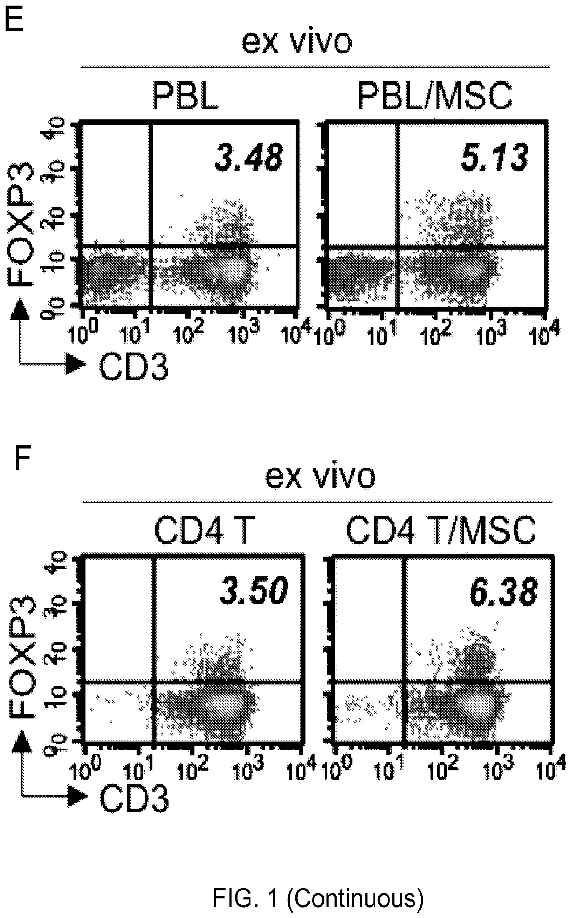

Figure 1. Human MSCs suppress IL-17A expression in in vitro expanded PBLs and CD4 cells, and augment FOXP3 expression in human T cells. 3 donors of human PBLs (A, representative data; B, pooled data) or CD4 T cells (C, representative data; D, pooled data) were co-cultured without (left panels) or with (right panels) hMSCs (donors A & B) upon anti-CD3/CD28 beads stimulation in vitro for 3 days. After phorbol 12-myristate 13-acetate (PMA)/ionomycin stimulation for 6 hours, IL-17A production in activated CD3+ T cells was assessed by intracellular staining. Numbers in the top right quadrants represent IL-17A+ cell percentages. *, p<0.05. Freshly isolated PBLs (E) or CD4 T cells (F) were cocultured without (left panels) or with (right panels) human MSCs with FOXP3 expression in CD3+ T cells assessed by intracellular staining. Representative dotplots are shown for experiments using 3 donors of PBLs/T cells and 2 donors of hMSCs; percentages of FOXP3+CD3+ T cells are shown in the top right quadrants.

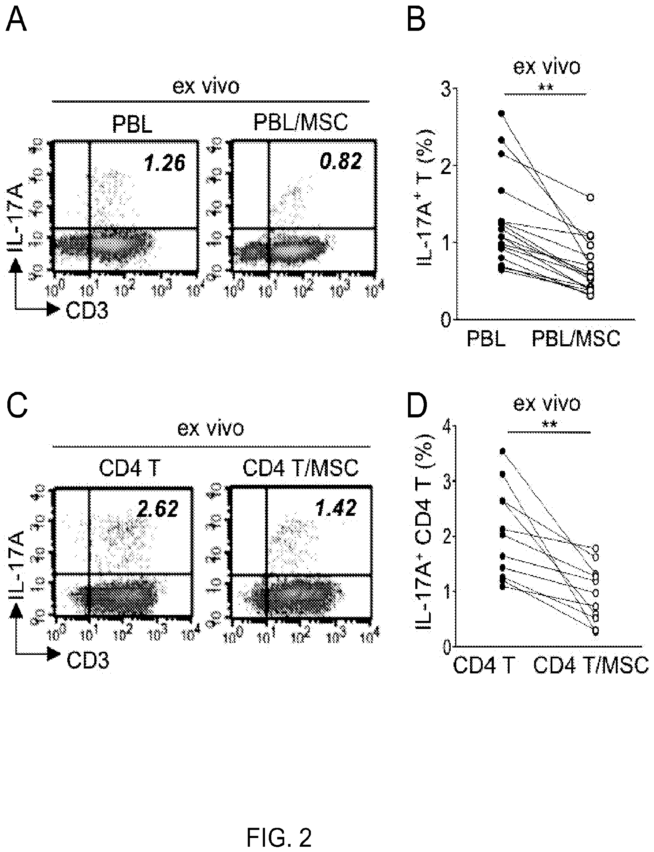

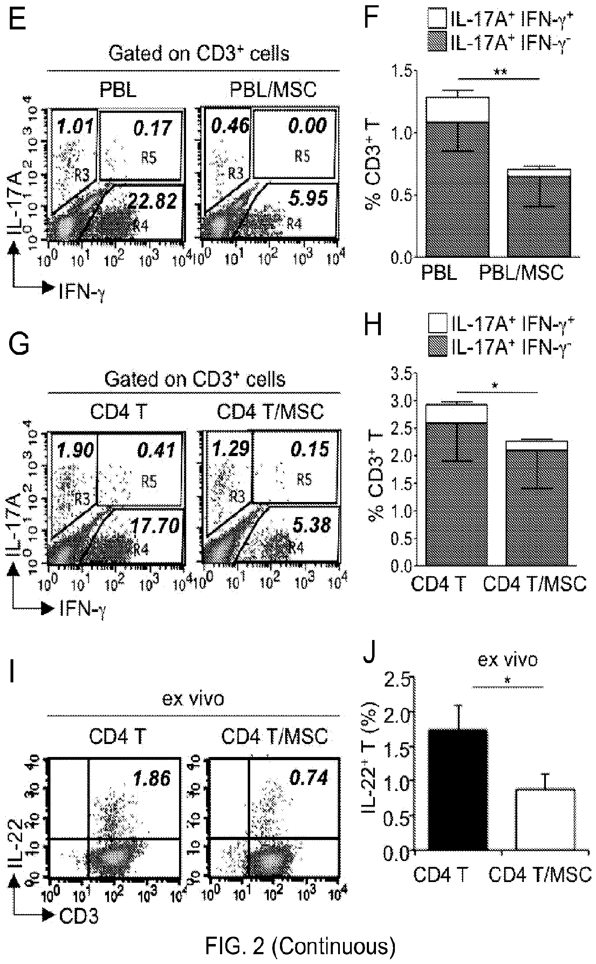

Figure 2. Multipotent human mesenchymal stromal cells (hMSCs) suppress Th17 responses. Human peripheral blood CD3⁺ leukocytes (PBLs) (A, representative data; B, pooled data) or CD3⁺ CD4 T cells (C, representative data; D, pooled data) were co-cultured without (left panels) or with (right panels) hMSCs *ex vivo,* followed by PMA/ionomycin stimulation for 6 hours. IL-17A production in *ex vivo* cultured CD3⁺ T cells was assessed by intracellular staining. Pooled data from PBLs (n=17) or CD4 T cells (n=11) co-cultured with all 3 hMSC donors are provided in (B) and (D), respectively. IL-17A and IFN-γ production in CD3⁺ PBLs (E, representative data; F, pooled data) or CD3⁺ CD4 T cells (G, representative data; H, pooled data) without and with co-culture of hMSCs was analyzed by flow cytometry. Representative intracellular staining is shown for IL-17A⁺ IFN-γ⁻-CD3⁺ T cells (R3 region) and IL-17A⁺IFN-γ⁺ (R5 region) CD3⁺ T cells and pooled data from PBLs (n=4) or CD4 T cells (n=4) co-cultured with 2 hMSC donors (donors A & B) are provided in (F) and (H), respectively. Gray bars represent percentage of IL-17A⁺ IFN-γ⁻-CD3⁺ T cells, whereas white bars represent the percentages of IL-17A⁺IFN-γ⁺ T cells. IL-22 production in 4 donors of CD3⁺ CD4 T cells (I, representative data; J, pooled data) without and with co-culture of 2 donors of hMSCs (donors A & B) was assessed by intracellular staining. Cell percentages are denoted in the dotplot quandrant of interest. Data are shown as mean ± SD; *,*p* < 0.05; ***,p* < 0.01.

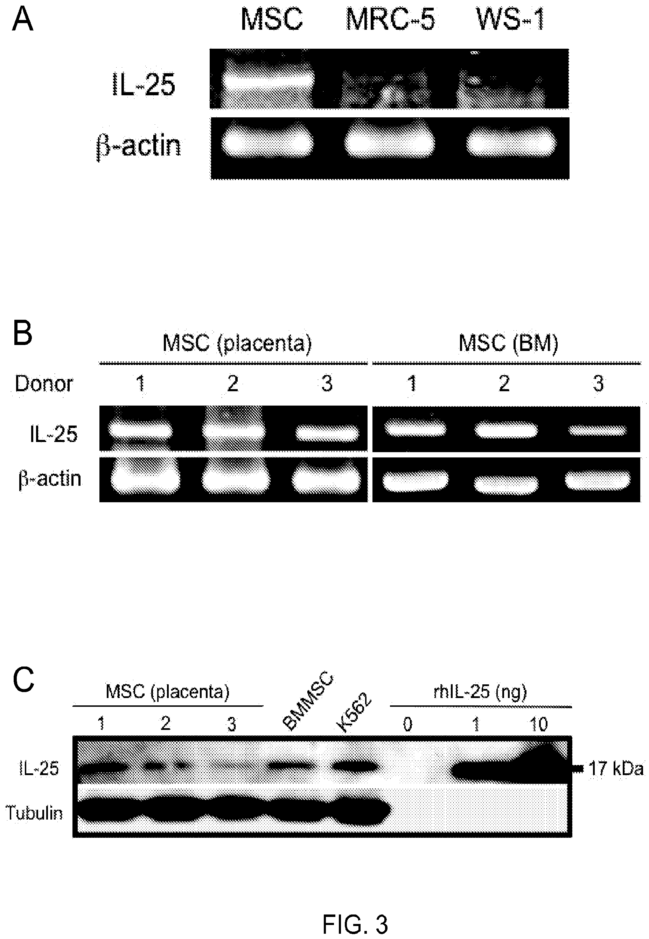

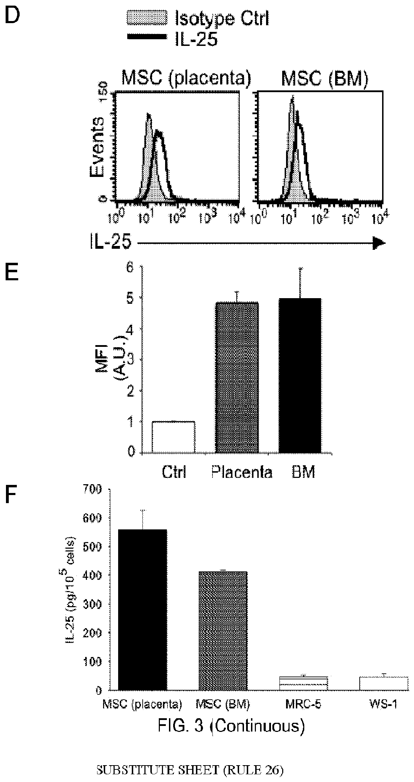

Figure 3. hMSCs constitutively express IL-25. Gene expression of IL-25 in placenta-derived hMSCs and fibroblast cell lines (MRC-5 and WS-1) (A), as well as 3 donors each of placental (left panel) and bone marrow (BM; right panel) hMSCs (B) was assessed by RT-PCR. Protein expression of IL-25 was determined by (C) western blotting (K562 cell line and indicated amounts of recombinant human IL-25 (rhIL-25) as positive controls; tubulin as internal control) and (D) intracellular staining for flow cytometric analysis (left panel, placental hMSCs donor A; right panel, BM hMSCs donor A). Filled histograms representing isotype control; unfilled histograms represent IL-25 antibody staining, with pooled data (3 donors each of placental and BM hMSCs) shown in (E). Ctrl, isotype control; MFI, mean fluorescence intensity; A.U., arbitrary units. (F) Secreted IL-25 by placental hMSCs (donor C, black bar), BM hMSCs (donor B, gray bar), MRC-5 (striped bar), or WS-1 (white bar) was assessed by collected conditioned medium of each cell type and analyzed by ELISA. Data are shown as mean ± SD of triplicates.

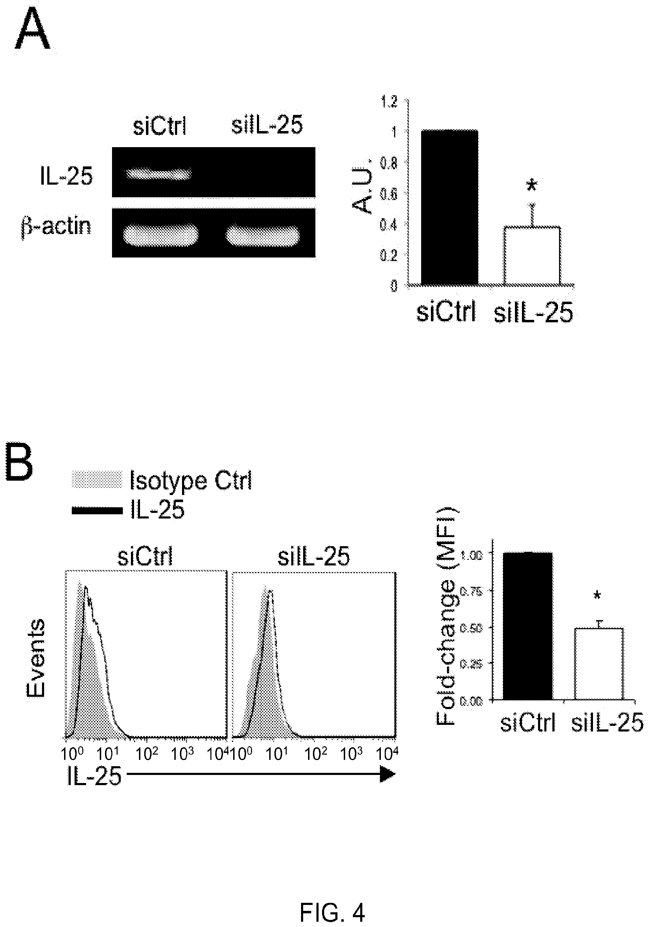

Figure 4. siIL-25 successfully silences the expression of IL-25 in human MSCs. IL-25 expression in MSCs with small interfering RNA for non-specific sequences (siCtrl) or IL-25 (siIL-25) was analyzed by (A) RT-PCR showing representative gel and quantitation by densitometry (all 3 donors); and (B) intracellular staining. Filled histograms represent isotype control. Unfilled histograms represent IL-25 antibody staining. A.U., arbitrary units; MFI, mean fluorescence intensity; *, p<0.05.

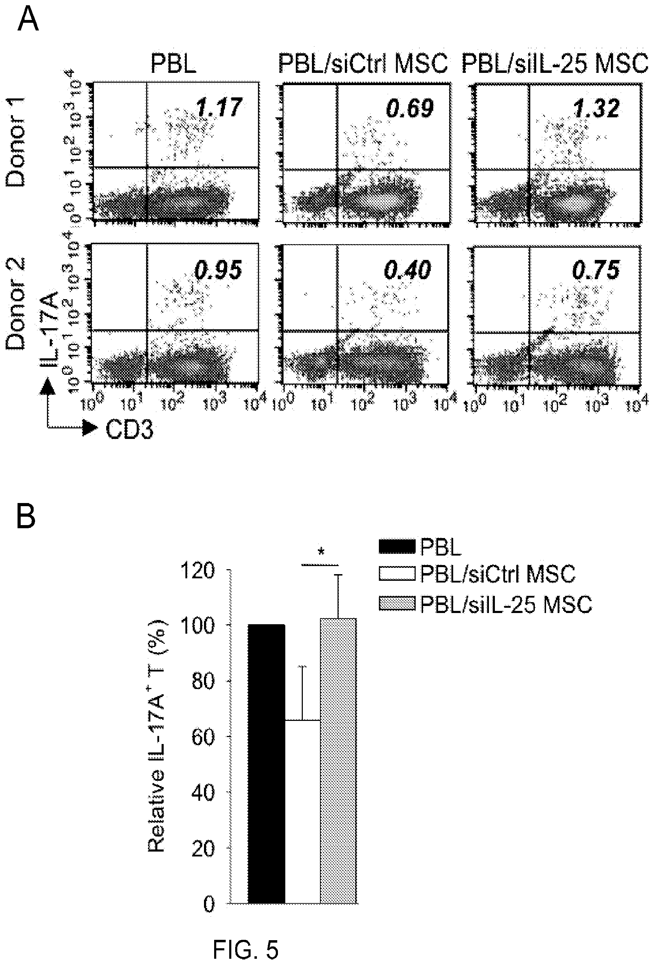

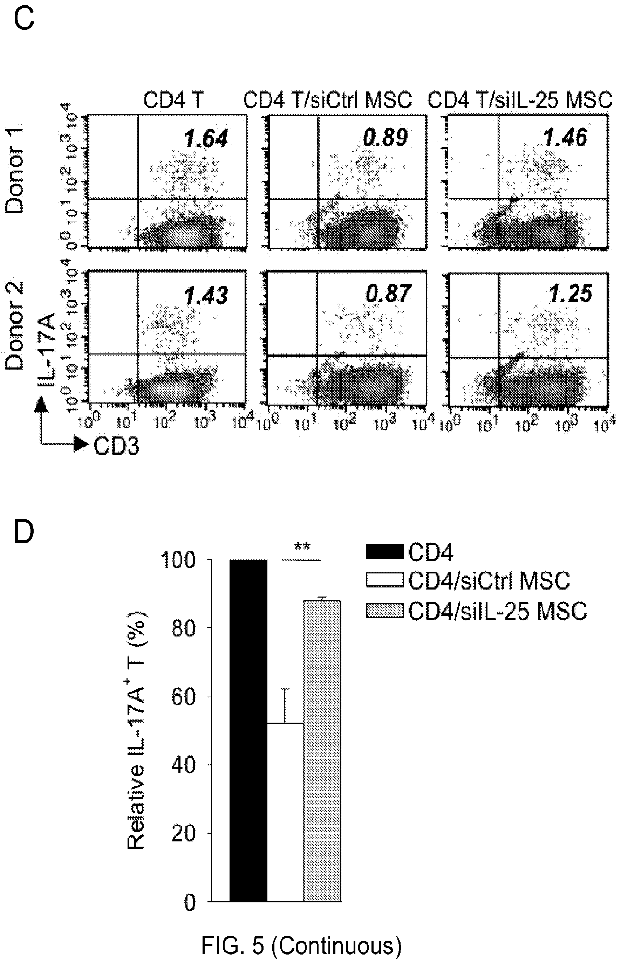

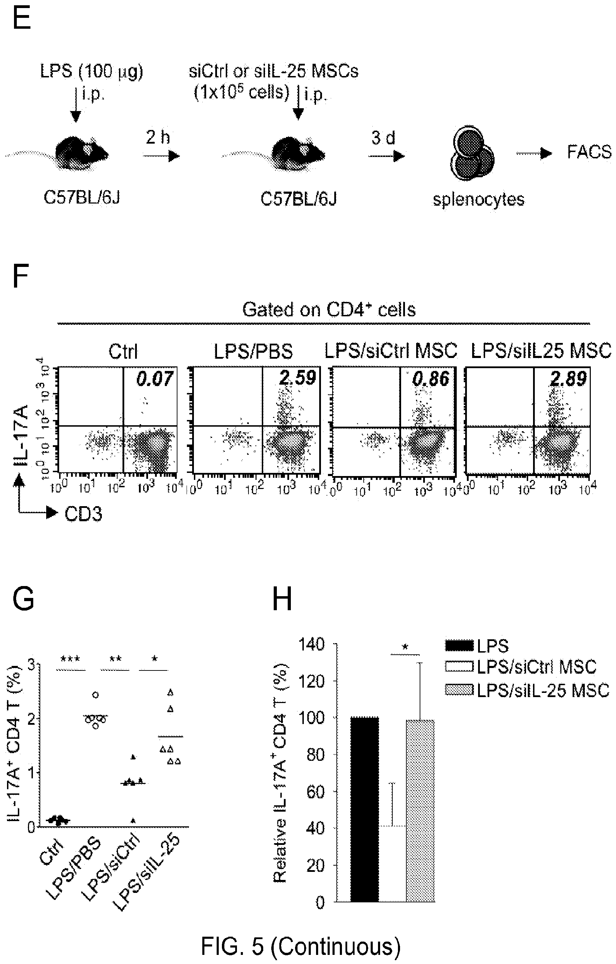

Figure 5. IL-25 silencing in hMSCs reverses Th17 responses *in vitro* and *in vivo.* Freshly isolated human PBLs (A) or CD4 T cells (C) were co-cultured without (left panels) or with either siCtrl hMSCs (middle panel) or siIL-25 hMSCs (right panel) for 3 days, followed by PMA/ionomycin stimulation for 6 hours. IL-17A production in CD3⁺ T cells was assessed by intracellular staining. Numbers in the top right quadrants represent the percentages of IL-17A-producing CD3⁺ T cells. Pooled data from PBLs (n=3) or CD4 T cells (n=3) and 2 hMSC donors (donors A & B) are provided in (B) and (D), respectively. Data are shown as mean ± SD. *, *p* <0.05; **, *p* < 0.01. (E) Experimental strategy for establishing in vivo inflammatory conditions in wildtype C57BL/6J mice with expansion of Th17 cells and adoptive transfer of hMSCs. (F) On day 3 after LPS (100 µg/mouse) challenge, IL-17A production in activated CD4 T cells in splenocytes from control mice, PBS-treated mice, siCtrl-hMSC-treated mice, or siIL-25-hMSC-treated mice was assessed by intracellular staining. Calculated (G) and relative (H) mean percentage of IL-17A-expressing CD4 T cells among control mice, PBS-treated mice, siCtrl-hMSC-treated mice, or siIL-25-hMSC-treated mice (n=6). Data are shown as mean ± SD. **,p* <0.05. ***,p* < 0.01; ****,p* < 0.005.

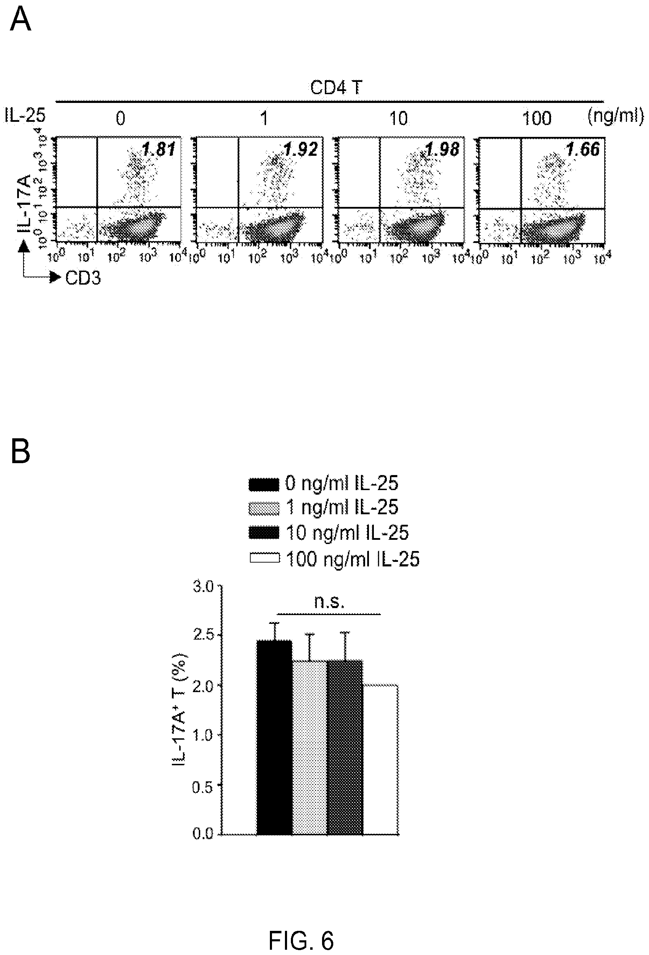

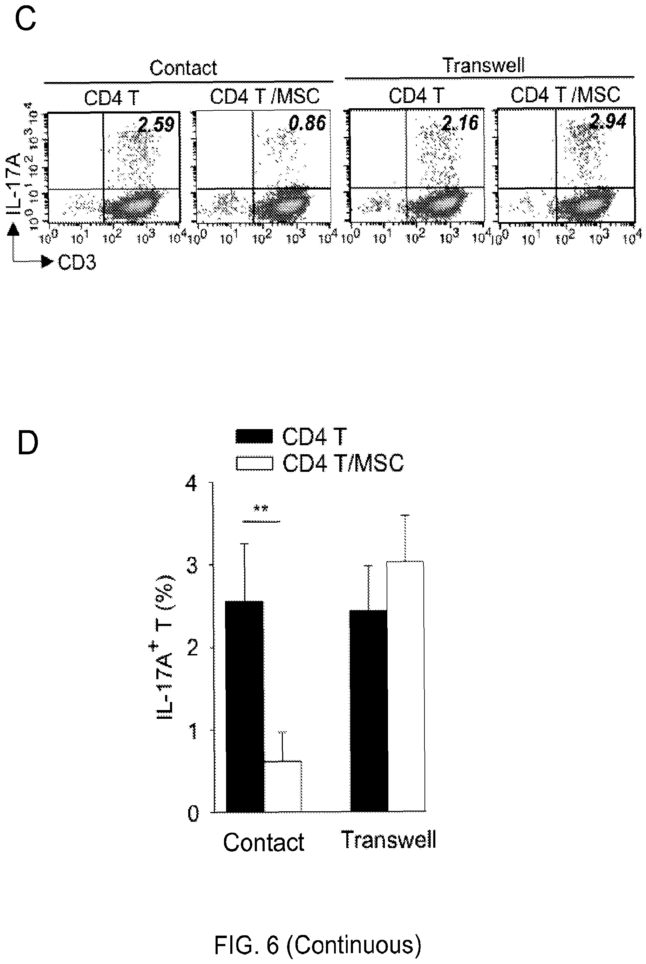

Figure 6. Exogenous IL-25 alone is insufficient to significantly suppress Th17 responses, with cell contact required as well for hMSC-mediated inhibition of Th17 responses. (A) Human CD4 T cells were treated with indicated doses of recombinant human IL-25 (rhIL-25) for 18 hours, followed by PMA/ionomycin stimulation for 6 hours. IL-17A production in CD3⁺ T cells was assessed by intracellular staining. Numbers in the top right quadrants represent the percentages of IL-17A-producing CD3⁺ T cells; (B) pooled data of 5 PBL donors. (C) Human CD4 T cells (n=4) were co-cultured without or with hMSCs (2 donors: B & C) in the absence or presence of transwell barriers. Pooled data from healthy donors are provided in (D). Data are shown as means ± SD. **,*p*<0.01; n.s., not significant.

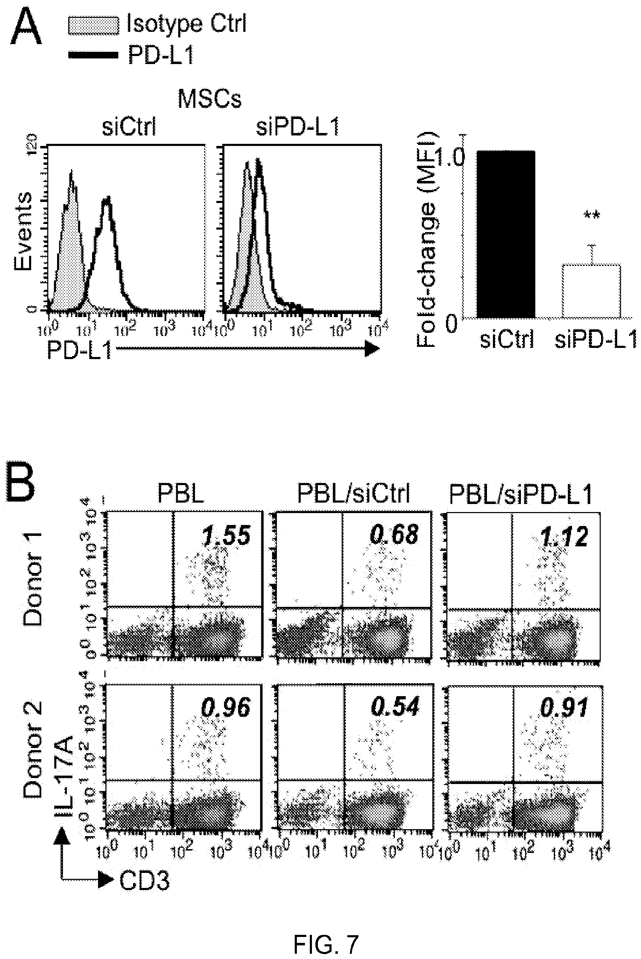

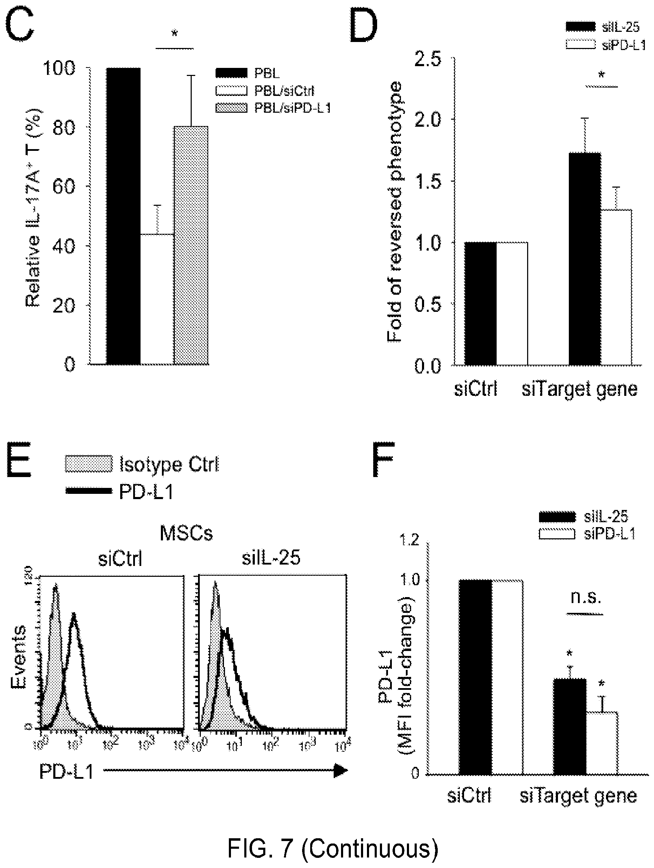

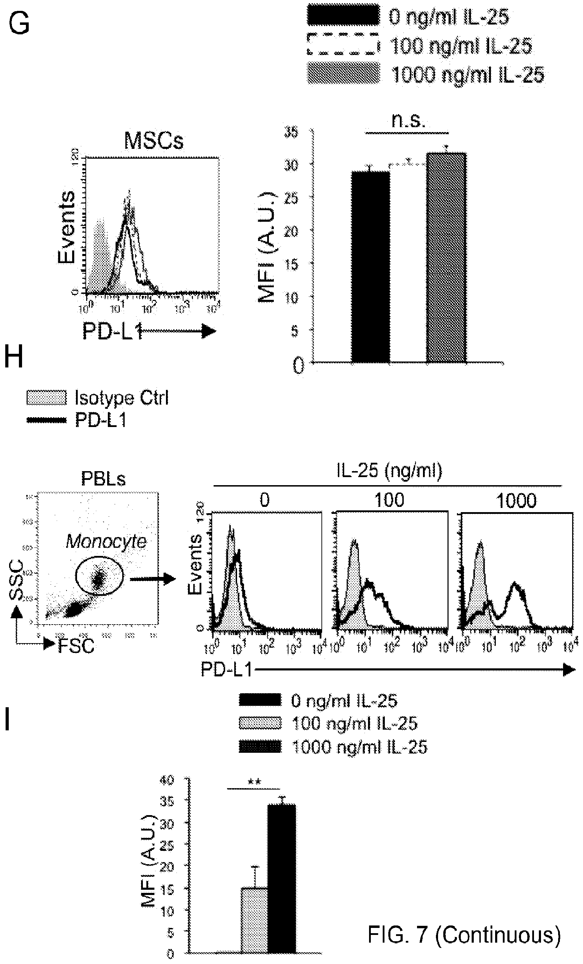

Figure 7. IL-25 induces PD-L1 surface expression on hMSCs and human monocytes. (A) PD-L1 in siCtrl MSCs (left panel) and siPD-L1 MSCs (right panel) was analyzed by surface staining. (B) Freshly isolated human PBLs were co-cultured without (left panels) or with siCtrl MSCs (middle panels) or siPD-L1 MSCs (right panels) for 3 days, followed by PMA/ionomycin stimulation for 6 hours. IL-17A production in CD3⁺ T cells was assessed by intracellular staining. Representative data shown with numbers in the top right quadrants representing the percentages of IL-17A-producing CD3⁺ T cells. Pooled data from PBLs (n=4) and 2 hMSC donors (donors A & B) are provided in (C). Folds of reversed phenotypes of siIL-25 and siPD-L1 are provided in (D). (E) PD-L1 expression on siCtrl hMSCs (left panel) and siIL-25 hMSCs (right panel) was assessed by cell surface staining. Filled histograms represent isotype control. Unfilled histograms represent PD-L1 antibody staining. Pooled data provided in (F) of PD-L1 expression (indicated by fold-change in mean fluorescence intensity; MFI) on siIL-25-hMSCs and siPD-L1-hMSCs (all 3 donors). PD-L1 expression levels were compared between hMSCs silenced for the target gene (IL-25 or PD-L1) to respective siCtrl. (G) hMSCs were treated with indicated doses of rhIL-25 for 18 hours and assessed for cell surface PD-L1 expression by cell surface staining. Pooled data (all 3 donors) shown in chart to the right with bars representing MFI (arbitrary units; A.U.). (H) Human PBLs were treated with indicated doses of rhIL-25 for 18 hours, and assessed for cell surface PD-L1 expression on monocytes, gated using FSC and SSC, by flow cytometric analysis. Pooled data (10 PBL donors) provided in (I) with bars representing MFI. *,*p*<0.05; **,*p*<0.01; n.s., not significant.

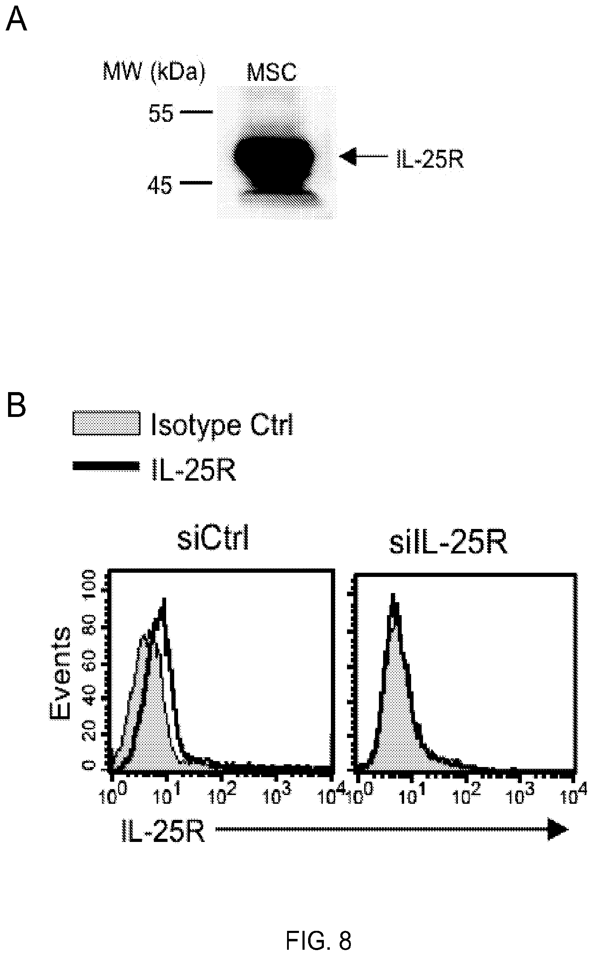

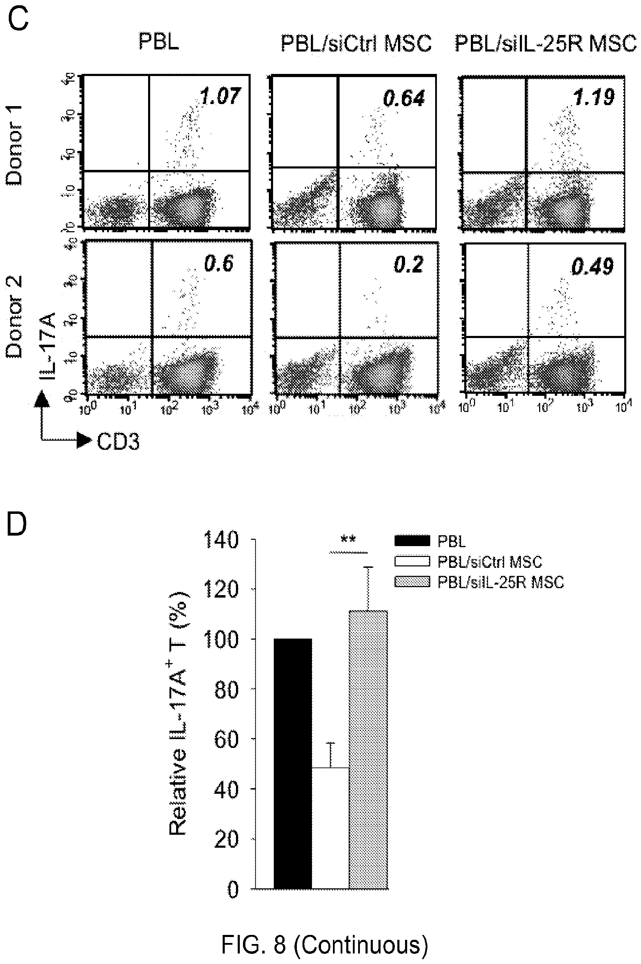

Figure 8. IL-25R silencing impairs the inhibition of IL-17A production in T cells by human MSCs. (A) IL-25R expression on human MSCs was determined by immunoblot. (B) IL-25R on siCtrl MSCs (left panel) and siIL-25R MSCs (right panel) was analyzed by surface staining. Filled histograms represent isotype control. Unfilled histograms represent IL-25R antibody staining. (C) Freshly isolated human PBLs were co-cultured without (left panels) or with siCtrl MSCs (middle panels) or siIL-25R MSCs (right panels; donors A & B) for 3 days, followed by PMA/ionomycin stimulation for 6 hours. IL-17A production in CD3+ T cells (3 donors) was assessed by intracellular staining. Numbers in the top right quadrants represent the percentages of IL-17A-producing CD3+ T cells. Pooled data from PBLs (n=3) is provided in (D).

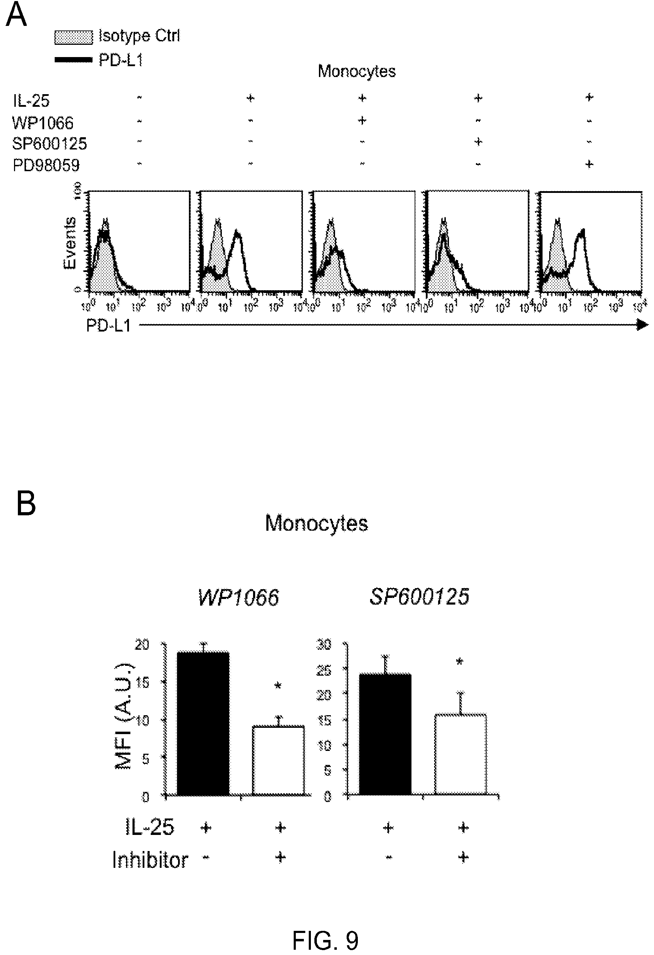

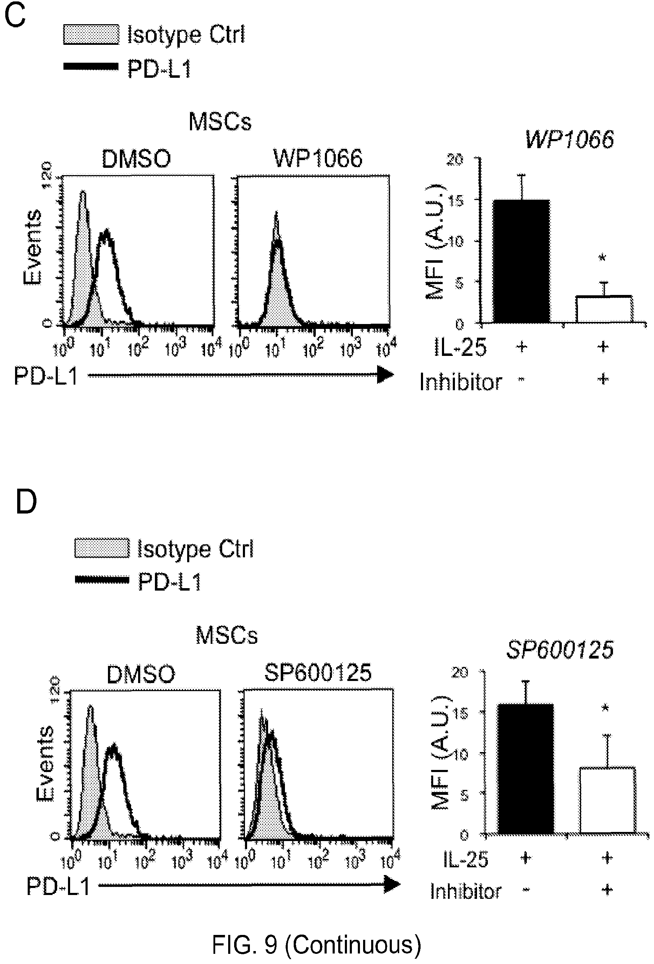

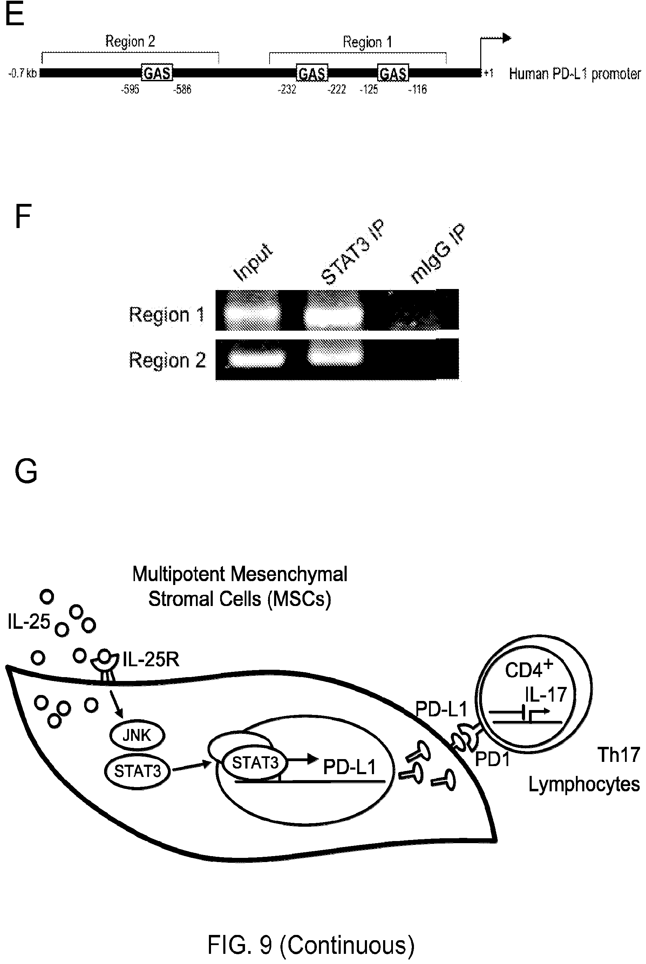

Figure 9. IL-25-mediated PD-L1 expression in human monocytes and hMSCs is mediated through JNK and STAT3 with STAT3 involved in transcriptional control of PD-L1. (A) Human PBLs were pretreated with inhibitors of STAT3 (WP1066; 2.5µM), JNK (SP600125; 25µM), or MEK1 (PD98059; 20µM) prior to 100 ng/ml rhIL-25 for 18 hours, with subsequent flow cytometric analysis for PD-L1 surface expression on monocytes, gated using FSC and SSC. Filled histograms represent isotype control. Unfilled histograms represent PD-L1 antibody staining. Pooled data (3 donors) provided in (B) with bars representing MFI. hMSCs were treated with inhibitors of (C) STAT3 (WP1066; 2.5µM) and (D) JNK (SP600125; 25µM) for 6 hours, and subsequently assessed by flow cytometric analysis for PD-L1 surface expression. Pooled data (all 3 donors) for each respective inhibitor provided (left charts) with bars representing MFI. (D) Putative GAS elements (STAT-binding sites) in the proximal promoter region of human PD-L1 gene; 700 bp region upstream from the transcription start site), as determined with TFSearch web-based software. (E) Binding of STAT3 or IgG (negative control) in hMSCs was analyzed by chromatin immunoprecipitation (ChIP) with promoter-specific primers for region 1 and region 2. The input samples (positive control) represent 1% starting chromatin. (F) Schematic of model of hMSC-mediated suppression of Th17 responses involving IL-25/STAT3/PD-L1 axis.

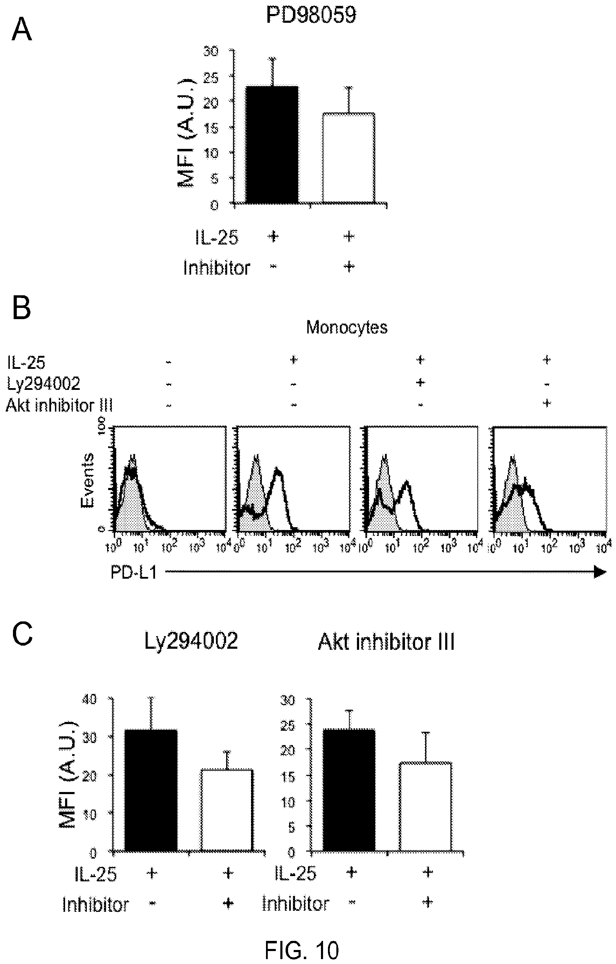

Figure 10. Involvement of signal pathways for IL-25-induced expression of PD-L1 in human peripheral blood monocytes. (A) Pooled data (3 donors) of PD-L1 expression (shown as MFI) after MEK inhibition. (B) Human peripheral blood monocytes were pretreated with inhibitors of PI3K (LY294002; 10µM) or Akt (Akt inhibitor III; 10µM), followed by 100 ng/ml IL-25 stimulation. After IL-25 treatment for 18 hours, the expression of PD-L1 was assessed by cell surface staining. Pooled data (3 donors) provided in (C) of PD-L1 expression (shown as MFI). A.U., arbitrary units.

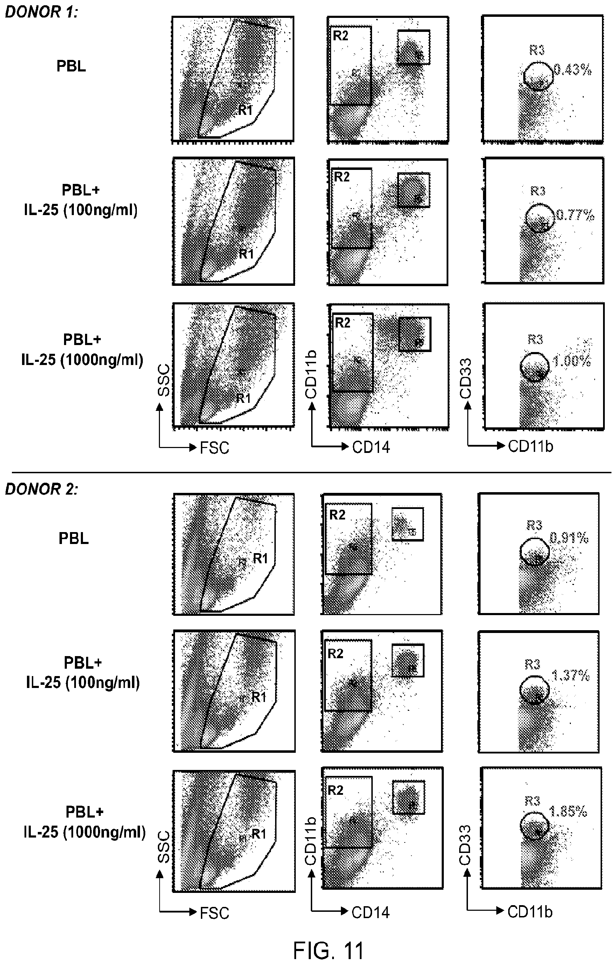

Figure 11. IL-25 expands CD33-/CD11b+/CD33+ myeloid-derived suppressor cells (MDSCs) in peripheral blood leukocytes (PBLs). Expansion of CD14⁻CD11b⁺CD33⁺ cells from human PBLs by recombinant human IL-25. Allogeneic PBL (2 donors) were cultured alone or with addition of various doses of IL-25 as indicated. PBL were first gated on forward scatter (FSC)/side scatter (SSC) (R1) then analyzed for CD14⁻ and CD11b⁺ (R2), and further analyzed for CD33⁺ (R3, cell percentages denoted).

### DETAILED DESCRIPTION OF THE EMBODIMENTS

In the present application, a method of upregulating an expression of immunomodulatory cells *in vitro* comprises treating the immunomodulatory cells with IL-25 to increase an expression of PD-L1, wherein the immunomodulatory cells are human monocytes or human mesenchymal stromal cells (hMSCs).

In some embodiments, the hMSCs can be isolated from a bone marrow, an adipose tissue, an umbilical cord blood, or a placenta. In another embodiment, the human monocytes can be isolated from peripheral blood leukocytes. In an embodiment, the method of upregulating an expression of immunomodulatory cells *in vitro* further comprises treating the immunomodulatory cells with the JNK inhibitor SP600215 and/or the STAT3 inhibitor WP1066.

In the present application, immunomodulatory cells selected from the group consisting of human monocytes and human MSCs, and IL-25, for use in the treatment of immune disorders. In some embodiment, the immune disorders can be autoimmune diseases, transplantation, and inflammatory diseases.

### Examples

### EXPERIMENTAL PROCEDURES

### Cell Culture

hMSCs from BM and placenta were isolated and expanded according to previous published protocols (Pittenger, M.F. et al., Science 1999, 284, 143-147; Yen, B.L. et al., Stem Cells 2005, 23, 3-9). Briefly, placenta MSCs were isolated from term human placentas (38- to 40-week gestation; 3 donors designated A, B, & C) obtained with informed consent approved by institutional review board. Placental tissue was mechanically and enzymatically digested (0.25% trypsin-EDTA; Gibco-Invitrogen) and cultured in Dulbecco's modified Eagle medium (DMEM)-low glucose (Gibco-Invitrogen), 10% fetal bovine serum (FBS; Hyclone), 2 mM L-glutamine (Gibco-Invitrogen), and 100 U/ml penicillin-streptomycin (Gibco-Invitrogen). BMMSCs were obtained commercially (Cambrex, 2 donors designated A & B; and Promocell, 1 donor designated C). All hMSCs used are placental-derived unless otherwise indicated. Human peripheral blood leukocytes (PBL) were isolated from the buffy coat of healthy donor blood samples (Taiwan Blood Services Foundation, Taipei Blood Center, Taipei, Taiwan) obtained with informed consent approved according to the procedures of the institutional review board and cultured as previously reported (Chang, C.J. et al., Stem Cells 2006, 24, 2466-2477; Yen, B.L. et al., Stem Cell Reports 2013, 1, 139-151). CD4 T cells were purified from PBL using human CD4 MicroBeads (Miltenyi Biotec) according to the manufacturer's protocols. Purity was assessed by flow cytometric analysis (>98% positive for CD4). Human fibroblast cell lines MRC-5 and WS-1 were obtained from American Type Culture Culture (ATCC) and cultured according to suggested protocols.

### MSC-leukocyte co-culture experiments

hMSCs were plated at 3.5x10⁴ cells per well in 6-well plates, and incubated at 37°C for 24 hours prior to co-culture with human PBL or CD4 cells. For co-cultures, 1x10⁵ human PBL or CD4 cells were added to hMSC-containing wells without or with stimulation by magnetic anti-CD3/CD28 coated Dynabeads (Gibco-Invitrogen), according to the manufacturer's instructions. After 3 days, cells were stimulated by phorbol 12-myristate 13-acetate (PMA) (50 ng/ml; Sigma-Aldrich) plus ionomycin (1 µg/ml; Sigma-Aldrich) in the presence of Monensin (eBioscience) for 6 hours, followed by assessment of IL-17A expression in T cells using intracellular staining. For transwell cultures, human PBL or CD4 cells were plated in the upper compartment of transwell plates (0.4 µm pore size; BD Falcon™), while hMSCs were plated in the lower compartment. Human recombinant IL-25 (rhIL-25; PeproTech) and various inhibitors (WP1066 /InSolution™ STAT3 Inhibitor III and Akt Inhibitor III from Millipore; SP600125 JNK inhibitor and LY294002 PI3 Kinase inhibitor from Cell Signaling Technology; and PD98059/MEK1/2 Inhibitor from Cell Signaling Technology) were added to various experiments at the indicated doses after establishing toxicity profiles for monocytes and hMSCs.

### Flow Cytometry

Cells were stained with antibodies as indicated: anti-human IL-25-PE (R&D Systems), mouse IgG1 isotype control-PE (R&D Systems), anti-human CD3-PE/Cy5 (BioLegend), anti-human CD4-PE (BioLegend), anti-human IL-17A-PE (eBioscience), anti-human IFN-γ-FITC (BioLegend), anti-human IL-22-PE (eBioscience), anti-human FOXP3-Alexa Fluor 488 (BD Pharmingen), anti-human CD274 (B1-H1)- PE (eBioscience), mouse IgG1 isotype control-PE (eBioscience), anti-human IL-25R-PE (R&D Systems), and mouse IgG2b isotype control-PE (R&D Systems), anti-mouse CD4-APC (eBioscience), anti-mouse CD3e-PE/Cy5 (eBioscience), and anti-mouse/rat IL-17A-PE (eBioscience). Data was collected on BD FACSCalibur™ (BD Biosciences) instruments and analyzed with Cell Quest Pro software (BD Biosciences).

### Mass Spectrometry

Tandem mass (MS/MS) experiments were performed as previously reported (Chang, W.C. et al., Int. J Proteomics 2010, 726968). Briefly, MS/MS was performed with an LTQ-FT ICR MS (Thermo Electron) equipped with a nanoelectrospray ion source (New Objective), an Agilent 1100 Series binary HPLC pump (Agilent Technologies) and a Famos autosampler (LC Packings). A minimum threshold of 1,000 counts was used as the cutoff for MS/MS sequential isolation by LTQ, with singly charged ions rejected for MS/MS sequencing.

### RT-PCR

Total RNA was prepared from cells using TRIzol reagent (Gibco-Invitrogen) according to the manufacturer's instructions. The first-strand cDNA was synthesized from the RNA using Improm-II reverse transcriptase (Promega). For PCR, cDNA was subjected to PCR using the following primer sets. IL-25, forward 5'-TTCCTACAGGT GGTTGCATTC-3', reverse 5'-CGCCTGTAGAAGACAGTCTGG-3' (Furuta, S. *et al., Sci. Transl. Med.* 2011, 3, 78ra31); β-actin, forward 5'-TGGCACCACACCTTCTACA ATGAGC-3', reverse 5'-GCACAGCTTCTCCTTAATGTCACGC -3'.

### ELISA

The human IL-25 ELISA kit was obtained from PeproTech and performed according to manufacturer's instructions. The detection range is 0∼2000 pg/ml.

### RNA Interference

Human IL-25, IL-25R or PD-L1 expression in hMSCs was silenced using Stealth RNA interference (RNAi) Duplex Oligonucleotides (Gibco-Invitrogen) according to manufacturer's instructions with non-target siRNA (medium GC duplex) used as control. Transfection of RNAi was done using Lipofectamine RNAiMAX (Gibco-Invitrogen), according to the manufacturer's instruction. Knockdown efficiency was confirmed by flow cytometry.

### hMSC Adoptive Transfer

All animal work was performed in accordance with protocols approved by the institutional Animal Care and Use Committee. Wild-type C57BL/6J mice were purchased from the National Laboratory Animal Center of Taiwan (Taipei, Taiwan). Induction of Th17 cells *in vivo* was performed similarly as previously reported (Shi, G. et al., J. Immunol. 2013, 191, 415-423). Briefly, LPS (100 µg; Escherichia coli 00041:B4; Sigma-Aldrich) was injected intraperitoneally into 8- to 12-week-old mice, followed 2 hours later by transfer of hMSCs (1x10⁵ cells/mouse) after non-target or IL-25 RNAi transfection (siCtrl or siIL-25, respectively). Mice were sacrificed on day 3 with harvesting of splenocytes for assessment of IL-17A⁺ expression in CD4 T cells.

### Chromatin Immunoprecipitation (ChIP)

ChIP assay was performed using the EZ-Zyme™ Chromatin Prep Kit (Millipore) and EZ-ChIP™ chromatin immunoprecipitation Kit (Millipore) according to the kit manufacturer's protocols. The digested chromatin was used for multiple immunoprecipitations with anti-STAT3 (124H6) mouse monoclonal antibody (Cell Signaling Technology) and normal mouse IgG. One percent of the reaction was removed as input chromatin. PCR detection was performed using the primer sets specific for the putative STAT3 binding sites in the human *CD274* promoter region. The sequences of the primer sets were forward 5'-AGGTGCGTTCAGATGTTGGC-3' and reverse 5'-TGCCCAAGGCAGCAAATCCAG-3', amplifying the segment from -337 bp to -118 bp, and forward 5'-TGACACCATCGTCTGTCATC-3' and 5'-GTCAGCAGCAGACCCATATG-3', amplifying the segment from -803 bp to -477 bp.

### Immunoblot analyses

Total cell lysates were prepared by lysing cells in lysis buffer (300 mM NaCl, 50 mM HEPES [pH 7.6], 1.5 mM MgCl₂, 10% glycerol, 1% Triton X-100, 10 mM NaPyrPO₄, 1 mM EGTA, 0.1 mM EDTA, 1 mM DTT, 1 mM PMSF, and 1 mM Na₄VO₃) at 4°C for 15 min. Lysates were first clarified by centrifugation at 12,000 × g for 20 min. Equal amounts of samples were resolved in 7% SDS-PAGE, followed by transferring to nitrocellulose (GE Healthcare) and blotting with anti-IL-25R antibody (GeneTex).

### Statistical Analyses

Student t test (two-tailed) was performed for statistical analysis between two groups, and ANOVA was performed for statistical analyses of multiple groups. Statistical significance was set at *p*< 0.05. All data were expressed as mean ± SD

### Results

### hMSCs Inhibit Th17 Responses

Since there have been discrepant reports on MSC-Th17 interactions, it is first set out to answer whether hMSCs enhance or suppress Th17 cell expansion. To determine this, placenta-derived hMSCs was used, which it has been previously demonstrated to be trilineage multipotent progenitors and immunomodulatory, similar to BMMSCs (Yen, B.L. et al., Stem Cells 2005, 23, 3-9; Chang, C.J. et al., Stem Cells 2006, 24, 2466-2477; Yen, B.L. et al., Stem Cell Reports 2013, 1, 139-151). These hMSCs were then co-cultured with human peripheral blood leukocytes (PBLs) or purified CD4 T cells in steady state for 3 days. Approximately 1 to 3% of non-primed T cells became IL-17A producers after phorbol 12-myristate 13-acetate (PMA)/ionomycin treatment for 6 hours, and it was found that when hMSCs were present, the frequency of IL-17A-expressing T cells was strongly decreased by 60%-65% in PBLs (Fig. 2A, representative data; 2B, pooled data) or CD4 T cells (Fig. 2C, representative data; Fig 2D, pooled data). To further confirm this phenomenon, stimulation of PBLs or T cells was performed *in vitro* with anti-CD3/CD28 beads and plus ionomycin to activate the Th17 effector phenotype (Santarlasci, V. et al., Immunity 2012, 36, 201-214). It was found that the frequency of *in vitro* expanded IL-17A-expressing PBLs (Fig. 1A, representative data; Fig. 1B, pooled data) and T cells (Fig. 1C, representative data; Fig. ID, pooled data) was significantly reduced as well when hMSCs were present. It is known that the lineages of Tregs and Th17 are linked, with one lineage chosen over another to maintain immune homeostasis (Weaver, C.T. et al., Nat. Rev. Immunol. 2009, 9, 883-889). Concomitantly, it was found that after co-culture with hMSCs, the frequency of FOXP3-expressing natural Tregs in PBLs and CD4 cells was increased (Figs. IE and 1F). hMSCs not only suppress IL-17A cells, but also prominently suppress IL-17A/IFN-γ double producer cells, which are the dominant subtype of Th17 cells at inflammatory sites (Annunziato, F. et al., J. Exp. Med. 2007, 204, 1849-1861; Zielinski, C.E. et al., Nature 2012, 484, 514-518). Previous reports have shown that hMSCs strongly suppress IFN-γ production a prototypical Th1 cytokine-in PBLs and T cells (Aksu, A.E. et al., Clin. Immunol. 2008, 127, 348-358; Aggarwal, S. et al., Blood 2005, 105, 1815-1822), and it was also found this to be true (Figs. 2E and 2G, respectively). Additionally, it was found that hMSCs substantially suppressed IFN-y/IL-17A-expressing T cells (for PBLs: Fig. 2E, representative data & Fig. 2F, pooled data; for CD4 cells: Fig. 2G, representative data & Fig. 2H, pooled data). Th17 cells are also known to produce IL-22 (Dong, C., Nat. Rev. Immunol. 2008, 8, 337-348), and co-culture of CD4 T cells with hMSCs also significantly decreased IL-22 production (Fig. 2I, representative data; Fig. 2J, pooled data). These results therefore demonstrate that hMSCs effectively suppress Th17 responses.

### hMSCs constitutively express IL-25

In the human system, MSC-T cell interactions have predominantly involved paracrine factors (Kim, N. et al., Ann. Hematol. 2013, 92, 1295-1308); therefore, to identify possible candidate secreted factors capable of suppressing Th17 responses, mass spectrometry (MS) analysis was performed on hMSC conditioned medium. Surprisingly, MS/MS studies revealed that IL-25, also known as IL17E and a potent suppressor of Th17 responses (Kleinschek, M.A. et al., J. Exp. Med. 2007, 204, 161-170; Zaph, C. et al., J. Exp. Med. 2008, 205, 2191-2198), was highly secreted by hMSCs. To reconfirm MS/MS results, we examined for transcripts of IL-25 in various sources of hMSCs against other stromal cell types such as fibroblasts. IL-25 messenger RNA (mRNA) could be detected in hMSCs but not in human fibroblast cell lines MRC-5 or WS-1 (Fig. 3A). It was also detected the expression of IL-25 mRNA in 3 different donors each of placenta-derived hMSCs and human BMMSCs (Fig. 3B), suggesting the reliability of IL-25 expression across the different sources of hMSCs. To ascertain protein production of IL-25, western blot was performed for overall protein production (Fig. 3C) and intracellular flow cytometric analysis to detect IL-25 (Fig. 3D) and could detect protein expression in both assays. Moreover, it was also found that hMSCs expressed IL-25 protein that can be detected in a secreted form in the conditioned medium as detected by ELISA (Fig. 3E). These findings indicate that hMSCs constitutively produce IL-25.

*Silencing of hMSC-derived IL-25 reverses suppression of Th17 responses in vitro and in vivo, but exogenous IL-25 alone is not sufficient to repress Th1*7 *responses* to assess whether hMSC-secreted IL-25 is involved in suppressing Th17 responses, IL-25 (siIL-25) expression in hMSCs was silenced by RNA interference. After confirming the efficiency of knockdown (Figs. 4A and 4B), it was found that silencing of IL-25 secretion with small interfering RNA (siRNA) specific for the gene in hMSCs almost completely reversed the suppressive effects towards Th17 cells compared with control silenced hMSCs (siCtrl) (Fig. 5A). An average decrease of 35% in IL-17A-expressing T cells is seen with co-culture of siCtrl hMSCs; this is completely abrogated when siIL-25 hMSCs are applied (Fig. 5B). A similar trend was seen when siCtrl or siIL-25 hMSCs were co-cultured with purified CD4 lymphocytes (Fig. 5C). Th17 lymphocytes are decreased to an average of 52% of baseline when siCtrl hMSCs are used in the co-culture, compared to 87% when siIL-25 hMSCs are applied (Fig. 5D). It was further confirmed the capacity of hMSC-derived IL-25 for Th17 suppression under *in vivo* inflammatory conditions by adoptive transfer of either siCtrl hMSCs or siIL-25 hMSCs into lipopolysaccharide (LPS)-treated C57BL/6J mice (Fig. 5E). It was found that *in vivo* transfer of siCtrl hMSCs suppressed the population of IL-17A-expressing CD4 T cells in the spleen whereas siIL-25 hMSCs failed to achieve that (Figs. 5F and 5G). With transfer of siCtrl hMSCs, IL-17A-expressing T cells are decreased to an average of 41% of baseline, but transfer of siIL-25 hMSCs nearly completely abrogates these effects with IL-17A-expressing T cells back at an average level of 98% (Fig. 5H). These data demonstrate that IL-25 secretion by hMSCs is involved in suppressing Th17 responses *in vitro* and *in vivo.*

To further ascertain the role of IL-25 in suppressing Th17 responses, CD4 T cells were treated with recombinant human IL-25 (rhIL-25) for 18 hours prior to PMA/ionomycin stimulation and examined for levels of IL-17A in CD4 cells. To our surprise, it was found that the addition of IL-25 singly to CD4 T cells failed to suppress Thl7 responses to a significant extent (Fig. 6A, representative data; Fig. 6B, pooled data). Moreover, when hMSCs were separated from CD4 T cells by Transwell membrane, suppressive effects towards Th17 cells in CD4 cells were lost (Fig. 6C, representative data; Fig. 6D pooled data). This indicates that a membrane-bound factor is likely involved in IL-25-mediated effects.

### hMSC-secreted IL-25 suppresses Th17 responses by upregulating surface expression of PD-L1

It has been reported that PD-L1 ligand, which is constitutively expressed on hMSC cell surfaces (Chang, C.J. et al., Stem Cells 2006, 24, 2466-2477; Stagg, J. et al., Blood 2006, 107, 2570-2577), is a strong inhibitor of IL-17A production in human T cells (Brown, J.A. et al., J. Immunol. 2003, 170, 1257-1266; Hirahara, K. et al., Immunity 2012, 36, 1017-1030). Hence, it was considered the possibility that hMSC-secreted IL-25 effects on Th17 responses may be mediated through interacting with this MSC-cell surface molecule. To ascertain previous reports of the suppressive effects of PD-L1 on Th17 cells, knockdown of PD-L1 expression on hMSCs was performed with siPD-L1 (Fig. 7A). In line with previous reports, it was found that PD-L1 knockdown in hMSCs reversed the suppression of Th17 cells (Figs .7B and 7C), but to a significantly lesser degree than that with siIL-25 (Fig. 7D). To assess the role of PD-L1 in IL-25-dependent suppression of Th17 responses, it was asked whether IL-25 is involved in the expression of PD-L1 on hMSCs. It was found that when IL-25 was silenced in hMSCs, surface expression of PD-L1 was strongly reduced, to a degree similar to knockdown with siRNA specific for itself (Fig. 7E, representative data; Fig. 7F, pooled data of siIL-25 vs. siPD-L1), suggesting that IL-25 may induce PD-L1 expression. The receptor for IL-25 is IL25R (Lee, J. et al., J. Biol. Chem. 2001, 276, 1660-1664), and it was searched for expression of IL25R in hMSCs; western blotting revealed that hMSCs constitutively express this receptor (Fig. 8A). When IL-25R expression was silenced on hMSCs with siIL-25R (Fig. 8B), it was found that hMSCs-mediated suppression of Th17 response was significantly abrogated (Figs. 8C & 8D). Thus, hMSC-secreted IL-25 requires interaction with its receptor IL-25R on hMSCs to lead to suppression of Th17 responses.

To further ascertain interactions of IL-25 on PD-L1 expression, rhIL-25 was added directly to hMSCs and assayed for further upregulation of PD-L1. It was found that exogenous rhIL-25 can further upregulate surface expression of PD-L1 on hMSCs but not to a significant extent (Fig. 7G), which may be due to the fact that PD-L1 is constitutively expressed at a high level on hMSCs and thereby masking further effects of rhIL-25. Thus, to further clarify the significance of IL-25 on PD-L1 expression, human primary monocytes from PBLs were used since these cells are known to respond to IL-25 as well as express low levels of PD-L1 at baseline (Caruso, R. et al., Blood 2009, 113, 3512-3519). It was found that when human PBLs are treated with rhIL-25, PD-L1 expression was dramatically and significantly increased in monocytes in a dose-dependent manner (Fig. 7H, representative data; Fig. 7I, pooled data). Thus, these findings demonstrate that IL-25 is involved in regulation of PD-L1 surface expression in both hMSCs and human monocytes.

### IL-25-induced upregulation of PD-L1 is mediated through JNK and STAT3, with STAT3 involved in transcriptional control of PD-L1

It is next sought to explore the signaling pathways by which IL-25 mediate expression of PD-L1. Monocytes from human PBLs were first used, in which the expression of PD-L1 is inducible rather than constitutive, to answer this question. In human primary monocytes, WP1066, a STAT3 inhibitor, or SP600125, a JNK inhibitor, substantially abolished IL-25-mediated induction of PD-L1 (Fig. 9A, representative data; Fig. 9B, pooled data). In contrast, PD98059, a MEK1/2 inhibitor, showed minimal effect while LY294002, a PI3K inhibitor, and Akt inhibitor III only partially affected IL-25-induced expression of PD-L1 (Figs. 10A and 10B). In hMSCs, which constitutively express high levels of PD-L1, it was also found that inhibition of STAT3 with WP1066 (Fig. 9C) or JNK with SP600125 (Fig. 9D) strongly reduced PD-L1 expression.

Since STAT3 is also a transcription factor, it was reasoned that this molecule may not only be involved in the signal pathway of IL-25-mediated PD-L1 expression, but also play a role in transcriptional control of PD-L1. To answer this question, the promoter of human PD-L1 gene between nucleotide -700 and nucleotide +1 for putative STAT3 binding elements was first analyzed. Based on software prediction, three putative GAS elements (STAT3 binding sites) between 595 and 116 bp upstream of the transcriptional start site were found (Fig. 9E), raising the possibility that STAT3 may directly bind to the promoter of PD-L1. To test this possibility, chromatin immunoprecipitation (ChIP) was performed to determine whether STAT3 binds to the PD-L1 promoter in hMSCs, and it was found that STAT3 was constitutively recruited to the GAS elements on the PD-L1 promoter (Fig. 9F). Thus, the data demonstrate that in hMSCs, IL-25 mediates cell surface expression of PD-L1 through JNK and STAT3, with the latter involved in the transcriptional control of PD-L1 (Fig. 9G).

To further confirm that IL-25 can induce expansion of MDSCs from peripheral blood, Allogeneic PBL (2 donors) were cultured alone or with addition of various doses of IL-25 as indicated (Figure 11). The treated cells were first gated on forward scatter (FSC)/side scatter (SSC) (R1) then analyzed for CD14⁻ and CD11b⁺ (R2), and further analyzed for CD33⁺ (R3, cell percentages denoted). The data demonstrate that IL-25 expands CD33-/CD11b+/CD33+ MDSCs PBLs.

### Discussion

hMSCs are known to be broadly immunomodulatory, and these effects are therapeutically relevant (Caplan, A.I. et al., Cell Stem Cell 2011, 9, 11-15; Le Blanc, K. et al., Nat. Rev. Immunol. 2012, 12, 383-396; Uccelli, A. et al., Nat. Rev. Immunol. 2008, 8, 726-736). Th17 cells are now known to be involved in the pathogenesis of a number of autoimmune and chronic inflammatory diseases (Miossec, P. et al., Nat. Rev. Drug Discov. 2012, 11, 763-776); hMSC interactions with this important population of leukocytes, however, have shown to be discrepant (Darlington, P.J. et al., Ann. Neurol. 2010, 68, 540-545; Ghannam, S. et al., J. Immunol. 2010, 185, 302-312; Gonzalez, M.A. et al., Arthritis Rheum. 2009, 60, 1006-1019; Tso, G.H. et al., Stem Cells 2010, 28, 939-954; Xu, J. et al., Blood 2012, 120, 3142-3151). The data reveals that the effects of hMSCs on Th17 cells are suppressive, and require both a paracrine factor-IL-25-as well as a cell surface molecule, PD-L1. Moreover, expression of PD-L1 in hMSCs is linked to IL-25 through IL-25R and further downstream through JNK and STAT3, the latter of which is involved in the transcriptional control of PD-L1. Th17 cells have been recognized as a contributor to transplant rejection through unknown mechanisms (Antonysamy, M.A. et al., J. Immunol. 1999, 162, 577-584; Faust, S.M. et al., J. Immunol. 2009, 183, 7297-7306). The data may shed some light on the mechanisms behind the strong therapeutic effects of hMSC therapy on related diseases (Bassi, E.J. et al., Diabetes 2012, 61, 2534-2545; Sun, L. et al., Stem Cells 2009, 27, 1421-1432; Zhou, B. et al., Clin. Immunol. 2011, 141, 328-337) and implicate a role for IL-25 agonists in ameliorating autoimmune/inflammatory diseases as well as transplant rejection.

IL-25 (IL-17E) is a member of the IL-17 family (Iwakura, Y. et al., Immunity 2011, 34, 149-162). However, unlike IL-17A or F the better known members of this interleukin family-which have direct roles in autoimmune and chronic inflammatory diseases, IL-25 actually appears to protect against IL-17A/Th17 and Th1 states (Caruso, R. et al., Gastroenterology 2009a, 136, 2270-2279). IL-25 deficient mice, in addition to promoting Th1 responses, have higher amount of IL-17A-expressing T cells and IFN-γ-expressing T cells in Th17-mediated experimental autoimmune encephalomyelitis (EAE), a model of human multiple sclerosis (Kleinschek, M.A. et al., J. Exp. Med. 2007, 204, 161-170). Interestingly, accumulating data demonstrate that IL-25 has another role in the immune system by promoting Th2 responses, preventing helminth infections (Fallon, P.G. et al., J. Exp. Med. 2006, 203, 1105-1116) as well as eosinophilic airway inflammation (Kim, M.R. et al., Blood 2002, 100, 2330-2340). To date, reported sources of IL-25 include immune cells such as T cells, macrophages, monocyte-derived dendritic cells, mast cells, eosinophils, and basophils, as well as non-immune cells such as epithelial and endothelial cells (Monteleone, G. et al., Cytokine & Growth Factor Rev. 2010, 21, 471-475). It was found that IL-25 to be highly and constitutively expressed by diverse sources of hMSCs but not fibroblasts. Moreover, the data show that IL-25 is directly responsible for hMSC suppression of allogeneic Th17 responses including decreasing the highly pathogenic IL-17A/IFN-γ⁺ cells, further demonstrating that IL-25 is broadly protective against Th17 and Th1 responses. It is interesting to speculate on other possible biological roles of IL-25 in hMSCs, given its high constitutive expression. Further studies are ongoing to evaluate whether this cytokine plays a role in hMSC proliferation and/or differentiation.

One of the striking findings of the present study is that IL-25 directly upregulates the surface molecule PD-L1 in both leukocyte-monocytes-and non-leukocyte populations-hMSCs. PD-L1 is strongly immunosuppressive, being an inhibitor of autologous T cell activation in several autoimmune diseases (Keir, M.E. et al., Annu. Rev. Immunol. 2008, 26, 677-704), and blockade of its receptor, PD-1, on T cells, can be very effective against cancer immmuosuppression as recently demonstrated (Topalian, S.L. et al., N. Engl. J. Med. 2012, 366, 2443-2454). Recently, a report has shown that mouse MSCs suppress Th17 responses through this pathway (Luz-Crawford, P. et al., PloS One 2012, 7, e45272). However, data in this report show that blockage of the PD-L1/PD-1 pathway only partially reverse mouse MSC suppression of Th17 responses, implicating other factors in this process. The data also demonstrate that silencing of PD-L1 results in partial reversal of hMSC suppression of Th17 responses, while silencing of IL-25 results in a significantly higher and nearly complete reversal of hMSC-mediated Th17 suppression (Fig. 7D). In addition, the degree of knockdown of PD-L1 expression was similar whether siRNA specific for IL-25 or PD-L1 was used (Fig. 7F). Moreover, it was found evidence that IL-25 can directly affect the transcription of PD-L1 in both hMSCs and human leukocytes through STAT3, which helps to resolve the question of PD-L1 transcriptional control (Sumpter, T.L. et al., Eur. J. Immunol. 2011, 41, 286-290; Wolfle, S.J. et al., Eur. J. Immunol. 2011, 41, 413-424). Critically, mouse MSCs do not express PD-L1 in steady state, whereas hMSCs constitutively express high level of PD-L1 (Stagg, J. et al., Blood 2006, 107, 2570-2577). It is important to note that while data from mouse systems are clearly important, in MSC immunobiology, results from mouse and human systems have at times have been conflicting (Eliopoulos, N. et al., Blood 2005, 106, 4057-4065; Le Blanc, K. et al., Lancet 2008, 371, 1579-1586), as is the case with PD-L1 expression. Based on the clinical response to hMSC therapy on various immune-related diseases, it appears that hMSCs exert strong immunomodulatory effects (Le Blanc, K. et al., Nat. Rev. Immunol. 2012, 12, 383-396), which is not always evident with mouse studies (Eliopoulos, N. et al., Blood 2005, 106, 4057-4065). Thus, to elucidate mechanisms involved in hMSC therapeutic applications, *in vitro* studies using hMSCs are still critical to conduct.

In summary, the findings demonstrate that hMSCs suppress Th17 responses, which require both the secreted factor IL-25 and IL-25-mediated upregulation of surface PD-L1. The downstream signaling pathways of JNK and STAT3 are involved in IL-25-regulation of PD-L1, with STAT3 implicated in the transcriptional control of PD-L1. In addition to the known roles of Th17 cells in autoimmune and chronic inflammatory diseases, recent studies also show the importance of Th17 cells in enhancing the efficacy of checkpoint immunotherapy (Lutz, E.R. *et al., Cancer Immunol. Res.* 2014, 2, 616-631). Modulation of IL-25, therefore, may have strong clinical implications since this cytokine can modulate PD-L1/PD-1 interactions and Th17 cells as well. The findings provide a better understanding of the crosstalk between hMSCs and Th17 cells, as well as highlight the IL-25/STAT3/PD-L1 axis as a candidate therapeutic target for relevant diseases.

## Claims

1. A method of upregulating an expression of immunomodulatory cells in vitro comprising treating the immunomodulatory cells selected from the group consisting of human monocytes and human mesenchymal stromal cells (hMSCs) with IL-25 to increase an expression of PD-L1.

2. The method of claim 1, wherein the hMSCs are isolated from a bone marrow, an adipose tissue, a umbilical cord blood, or a placenta.

3. The method of claim 1, wherein the human monocytes are from peripheral blood leukocytes.

4. Immunomodulatory cells selected from the group consisting of human monocytes and human MSCs, and IL-25, for use in the treatment of immune disorders.

5. The immunomodulatory cells for the use of claim 4, wherein the immune disorders comprise autoimmune diseases, transplantation, and inflammatory diseases.

6. Immunomodulatory cells selected from the group consisting of human monocytes and human MSCs, IL-25 and a STAT3 inhibitor and/or a JNK inhibitor, for use in the treatment of immune disorders, wherein the STAT3 inhibitor is WP1066 and the JNK inhibitor is SP600125.

7. The immunomodulatory cells for the use of claim 6, wherein the immune disorders comprise autoimmune diseases, transplantation, and inflammatory diseases.

## Patentansprüche

1. Verfahren zum Hochregulieren einer Expression von immunomodulatorischen Zellen in vitro, umfassend Behandeln der immunomodulatorischen Zellen ausgewählt aus der Gruppe bestehend aus menschlichen Monozyten und menschlichen mesenchymalen Stromazellen (hMSCs) mit IL-25, um eine Expression von PD-L1 zu erhöhen.

2. Verfahren nach Anspruch 1, wobei aus Knochenmark, Fettgewebe, Nabelschnurblut oder Plazenta, die hMSCs isoliert wurden.

3. Verfahren nach Anspruch 1, wobei die menschlichen Monozyten aus peripheren Blutleukozyten bestehen.

4. Immunomodulatorische Zellen ausgewählt aus der Gruppe bestehend aus menschlichen Monozyten und menschlichen MSCs und IL-25 zur Verwendung in der Behandlung von immunologischen Störungen.

5. Immunomodulatorische Zellen zur Verwendung nach Anspruch 4, wobei die immunologischen Störungen, Autoimmunerkrankungen, Transplantation und entzündliche Erkrankungen umfassen.

6. Immunomodulatorische Zellen ausgewählt aus der Gruppe bestehend aus menschlichen Monozyten und menschlichen MSCs, IL-25 und einem STAT3-Inhibitor und/oder einem JNK-Inhibitor zur Verwendung in der Behandlung von immunologischen Störungen, wobei der STAT3-Inhibitor WP1066 ist und der JNK-Inhibitor SP600125 ist.

7. Immunomodulatorische Zellen zur Verwendung nach Anspruch 6, wobei die immunologischen Störungen Autoimmunerkrankungen, Transplantation und entzündliche Erkrankungen umfassen.

## Revendications

1. Procédé de régulation à la hausse d'une expression de cellules immunomodulatrices in vitro comprenant le traitement des cellules immunomodulatrices sélectionnées dans le groupe constitué de monocytes humains et de cellules stromales mésenchymateuses humaines (CSMh) par IL-25 pour augmenter une expression de PD-L1.

2. Procédé selon la revendication 1, les CSMh étant isolées à partir d'une moelle osseuse, d'un tissu adipeux, d'un sang de cordon ombilical ou d'un placenta.

3. Procédé selon la revendication 1, les monocytes humains provenant de leucocytes du sang périphérique.

4. Cellules immunomodulatrices choisies dans le groupe constitué de monocytes humains et de CSM humaines et d'IL-25, pour une utilisation dans le traitement de troubles immuns.

5. Cellules immunomodulatrices pour l'utilisation selon la revendication 4, les troubles immuns comprenant les maladies auto-immunes, une transplantation et les maladies inflammatoires.

6. Cellules immunomodulatrices choisies dans le groupe constitué de monocytes humains et de CSM humaines, d'IL-25 et d'un inhibiteur de STAT3 et/ou d'un inhibiteur de JNK, pour une utilisation dans le traitement de troubles immuns, l'inhibiteur de STAT3 étant WP1066 et l'inhibiteur de JNK étant SP600125.

7. Cellules immunomodulatrices pour l'utilisation selon la revendication 6, les troubles immuns comprenant les maladies auto-immunes, une transplantation et les maladies inflammatoires.


### SEQUENCE LISTING - Rule 13ter

<110> National Health Research Institute
   Wang, Lu-Hai
<120> METHODS TO UPREGULATE AND SUPPRESS AN EXPRESSION OF IMMUNOMODULATORY CELLS
<130> 76474.2021PCT
<140> PCT/US2015/047025
   <141> 2015-08-26
<150> US 62/052, 083
   <151> 2014-09-18
<160> 8
<170> PatentIn version 3.5
<210> 1
   <211> 21
   <212> DNA
   <213> Artificial Sequence
<220>
   <223> PCR primer
<400> 1
   ttcctacagg tggttgcatt c 21
<210> 2
   <211> 21
   <212> DNA
   <213> Artificial Sequence
<220>
   <223> PCR primer
<400> 2
   cgcctgtaga agacagtctg g 21
<210> 3
   <211> 25
   <212> DNA
   <213> Artificial sequense
<220>
   <223> PCR primer
<400> 3
   tggcaccaca ccttctacaa tgagc 25
<210> 4
   <211> 25
   <212> DNA
   <213> Artificial Sequence
<220>
   <223> PCR primer
<400> 4
   gcacagcttc tccttaatgt cacgc 25
<210> 5
   <211> 20
   <212> DNA
   <213> Artificial Sequence
<220>
   <223> PCR primer
<400> 5
   aggtgcgttc agatgttggc 20
<210> 6
   <211> 21
   <212> DNA
   <213> Artificial Sequence
<220>
   <223> PCR primer
<400> 6
   tgcccaaggc agcaaatcca g 21
<210> 7
   <211> 20
   <212> DNA
   <213> Artificial Sequence
<220>
   <223> PCR primer
<400> 7
   tgacaccatc gtctgtcatc 20
<210> 8
   <211> 20
   <212> DNA
   <213> Artificial Sequence
<220>
   <223> PCR primer
<400> 8
   gtcagcagca gacccatatg 20